# EUROPEAN PATENT APPLICATION

(11) **EP 1 254 667 A1**
(43) Date of publication of application: **06.11.2002**
(21) Application number: 01904344.7
(22) Date of filing: 08.02.2001
(51) Int. Cl.: A61K 45/06, A61K 31/22, A61K 31/366, A61K 31/40, A61K 31/427, A61K 31/44, A61K 31/4439, A61P 1/04, A61P 19/02, A61P 29/00, C07D 263/44

(54) **DRUG COMPRISING COMBINATION**

(30) Priority: 10.02.2000 JP 2000038265
(71) Applicant: Takeda Chemical Industries, Ltd., Osaka-shi, Osaka 541-0045 (JP)
(72) Inventor: SUGIYAMA, Yasuo;, 6-0111; (JP); ODAKA, Hiroyuki;, 651-1223; (JP); NARUO, Ken-ichi;, 5; (JP)
(74) Representative: Wright, Robert Gordon McRae
(86) International application number: JP0100880
(87) International publication number: WO01058491

(57) **Abstract**

A TNF-α inhibitor comprising an insulin sensitizer in combination with an HMG-CoA reductase inhibitor is useful as an agent for the prophylaxis or treatment of an inflammatory disease and the like.

## Description

### Technical Field

The present invention relates to a TNF-α inhibitor comprising an insulin sensitizer in combination with an HMG-CoA reductase inhibitor, which is useful as an agent for the prophylaxis or treatment of diseases in which TNF-α is involved, such as inflammatory disease and the like.

### Background Art

TNF (tumor necrosis factor)-α is considered to play an important role in various diseases. In rheumatoid arthritis, which is an inflammatory disease, for example, production of TNF-α is considered to be promoted, and this causes destruction of the articular tissues.

As regards combination of an insulin sensitizer and an HMG-CoA reductase inhibitor, the following have been reported.
1) JP-A-9-71540 (EP-A-753298) describes the use of one or more HMG-CoA reductase inhibitory substances and one or more insulin sensitizers, for the prophylaxis and/or treatment of arteriosclerosis and/or xanthoma.
2) JP-A-9-67271 (EP-A-749751) describes a pharmaceutical agent which comprises an insulin sensitizer in combination with at least one member of the group consisiting of an α-glucosidase inhibitor, an aldose reductase inhibitor, biguanides, a statin compound, a squalene synthesis inhibitor, a fibrate compound, an LDL catabolism enhancer and an angiotensin converting enzyme inhibitor.

However, neither of the above-mentioned publications describe nor suggest a TNF-α inhibitory effect.

Thus, there is a demand for the development of a TNF-α inhibitor having properties sufficiently superior as a pharmaceutical agent, such as a superior prophylaxis and/or treatment effect on diseases in which TNF-α is involved, such as inflammatory disease and the like, without side effects and the like.

### Disclosure of the Invention

The present invention relates to
(1) a TNF-α inhibitor comprising an insulin sensitizer in combination with an HMG-CoA reductase inhibitor;
(2) the inhibitor of the aforementioned (1), wherein the insulin sensitizer is a compound of the formula [I] 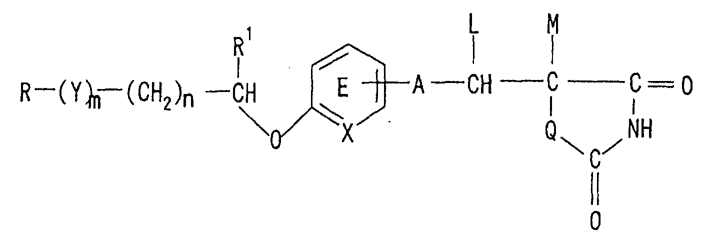 wherein
   - R: is an optionally substituted hydrocarbon group or an optionally substituted heterocyclic group;
   - Y: is a group represented by -CO-, -CH(OH)- or -NR³- wherein R³ is an optionally substituted alkyl group;
   - m: is 0 or 1;
   - n: is 0, 1 or 2;
   - X: is CH or N;
   - A: is a bond or a divalent aliphatic hydrocarbon group having 1 to 7 carbon atoms;
   - Q: is an oxygen atom or a sulfur atom;
   - R¹: is a hydrogen atom or an alkyl group;
   - ring E: may further have 1 to 4 substituents, wherein the substituent(s) may be bonded to R¹ to form a ring; and
   - L and M: are each a hydrogen atom or may form a bond in combination,
or a salt thereof;
(3) the inhibitor of the aforementioned (2), wherein the compound of the formula [I] is pioglitazone;
(4) the inhibitor of the aforementioned (2), wherein the compound of the formula [I] is rosiglitazone;
(5) the inhibitor of the aforementioned (1), wherein the HMG-CoA reductase inhibitor is a statin compound;
(6) the inhibitor of the aforementioned (1), wherein the HMG-CoA reductase inhibitor is cerivastatin, pravastatin, simvastatin, lovastatin, atorvastatin, fluvastatin, itavastatin, ZD-4522 or a salt thereof;
(7) the inhibitor of the aforementioned (1), wherein the HMG-CoA reductase inhibitor is atorvastatin or a salt thereof;
(8) the inhibitor of the aforementioned (1), which comprises pioglitazone or a salt thereof in combination with cerivastatin or a salt thereof;
(9) the inhibitor of the aforementioned (1), which comprises pioglitazone or a salt thereof in combination with pravastatin or a salt thereof;
(10) the inhibitor of the aforementioned (1), which comprises pioglitazone or a salt thereof in combination with atorvastatin or a salt thereof;
(11) the inhibitor of the aforementioned (1), which comprises rosiglitazone or a salt thereof in combination with cerivastatin or a salt thereof;
(12) the inhibitor of the aforementioned (1), which comprises rosiglitazone or a salt thereof in combination with pravastatin or a salt thereof;
(13) the inhibitor of the aforementioned (1), which comprises rosiglitazone or a salt thereof in combination with atorvastatin or a salt thereof;
(14) the inhibitor of the aforementioned (1), which is an agent for the prophylaxis or treatment of an inflammatory disease;
(15) the inhibitor of the aforementioned (14), wherein the inflammatory disease is rheumatoid arthritis;
(16) the inhibitor of the aforementioned (14), wherein the inflammatory disease is inflammatory bowel disease;
(17) a method for treating an inflammatory disease, which comprises administering an effective amount of an insulin sensitizer in combination with an HMG-CoA reductase inhibitor to a mammal;
(18) use of an insulin sensitizer for the production of a therapeutic agent of an inflammatory disease which is used in combination with an HMG-CoA reductase inhibitor; and the like.

The insulin sensitizer to be used in the present invention is a pharmaceutical agent that affords recovery from functional disorders of insulin receptor, and improves insulin resistance, which is exemplified by a compound having a thiazolidinedione or oxazolidinedione structure, preferably a compound represented by the aforementioned formula [I] and a salt thereof.

The hydrocarbon group of the optionally substituted hydrocarbon group represented by R in the formula [I] is exemplified by an aliphatic hydrocarbon group, an alicyclic hydrocarbon group, an alicyclic-aliphatic hydrocarbon group, an aromatic-aliphatic hydrocarbon group and an aromatic hydrocarbon group. These hydrocarbon groups preferably have 1 to 14 carbon atoms.

As the aliphatic hydrocarbon group, an aliphatic hydrocarbon group having 1 to 8 carbon atoms is preferable. As the aliphatic hydrocarbon group, for example, a saturated aliphatic hydrocarbon group (e.g., alkyl group etc.) having 1 to 8 carbon atoms, such as methyl, ethyl, propyl, isopropyl, butyl, isobutyl, sec.-butyl, t.-butyl, pentyl, isopentyl, neopentyl, t.-pentyl, hexyl, isohexyl, heptyl, octyl and the like; an unsaturated aliphatic hydrocarbon group (e.g., alkenyl group, alkadienyl group, alkynyl group, alkadiynyl group etc.) having 2 to 8 carbon atoms, such as vinyl, 1-propenyl, 2-propenyl, 1-butenyl, 2-butenyl, 3-butenyl, 2-methyl-1-propenyl, 1-pentenyl, 2-pentenyl, 3-pentenyl, 4-pentenyl, 3-methyl-2-butenyl, 1-hexenyl, 3-hexenyl, 2,4-hexadienyl, 5-hexenyl, 1-heptenyl, 1-octenyl, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 3-hexynyl, 2,4-hexadiynyl, 5-hexynyl, 1-heptynyl, 1-octynyl and the like are mentioned.

As the alicyclic hydrocarbon group, an alicyclic hydrocarbon group having 3 to 7 carbon atoms is preferable. Examples of the alicyclic hydrocarbon group include a saturated alicyclic hydrocarbon group (e.g., cycloalkyl group etc.) having 3 to 7 carbon atoms, such as cyclopropyl, cyclobutyl, cyclopentyl, cyclohexyl, cycloheptyl and the like and an unsaturated alicyclic hydrocarbon group (e.g., cycloalkenyl group, cycloalkadienyl group etc.) having 5 to 7 carbon atoms, such as 1-cyclopentenyl, 2-cyclopentenyl, 3-cyclopentenyl, 1-cyclohexenyl, 2-cyclohexenyl, 3-cyclohexenyl, 1-cycloheptenyl, 2-cycloheptenyl, 3-cycloheptenyl, 2,4-cycloheptadienyl and the like.

As the alicyclic-aliphatic hydrocarbon group, one
wherein the above-mentioned alicyclic hydrocarbon group and the aliphatic hydrocarbon group are bonded (e.g., cycloalkyl-alkyl group, cycloalkenyl-alkyl group etc.) is exemplified, of which an alicyclic-aliphatic hydrocarbon group having 4 to 9 carbon atoms is preferable. As the alicyclic-aliphatic hydrocarbon group, for example, cyclopropylmethyl, cyclopropylethyl, cyclobutylmethyl, cyclopentylmethyl, 2-cyclopentenylmethyl, 3-cyclopentenylmethyl, cyclohexylmethyl, 2-cyclohexenylmethyl, 3-cyclohexenylmethyl, cyclohexylethyl, cyclohexylpropyl, cycloheptylmethyl, cycloheptylethyl and the like are exemplified.

As the aromatic-aliphatic hydrocarbon group, an aromatic-aliphatic hydrocarbon group (e.g., aralkyl group etc.) having 7 to 13 carbon atoms is preferable. Examples of the aromatic-aliphatic hydrocarbon group include a phenylalkyl having 7 to 9 carbon atoms, such as benzyl, phenethyl, 1-phenylethyl, 3-phenylpropyl, 2-phenylpropyl, 1-phenylpropyl and the like, a naphthylalkyl having 11 to 13 carbon atoms, such as α-naphthylmethyl, α-naphthylethyl, β-naphthylmethyl, β-naphthylethyl and the like, and the like.

As the aromatic hydrocarbon group, an aromatic hydrocarbon group (e.g., aryl group etc.) having 6 to 14 carbon atoms is preferable. Examples of the aromatic hydrocarbon group include phenyl, naphthyl (α-naphthyl, β-naphthyl) and the like.

The heterocyclic group of the optionally substituted heterocyclic group represented by R in the formula [I] is, for example, a 5-7 membered heterocyclic group or fused ring group having, besides carbon atom, 1 to 4 heteroatoms selected from oxygen atom, sulfur atom and nitrogen atom as a ring-constituting atom. Examples of the fused ring is a fused ring of such 5-7 membered heterocyclic ring with a 6-membered ring containing 1 or 2 nitrogen atoms, a benzene ring or a 5-membered ring having one sulfur atom.

Specific examples of the heterocyclic group include 2-pyridyl, 3-pyridyl, 4-pyridyl, 2-pyrimidinyl, 4-pyrimidinyl, 5-pyrimidinyl, 6-pyrimidinyl, 3-pyridazinyl, 4-pyridazinyl, 2-pyrazinyl, 2-pyrrolyl, 3-pyrrolyl, 2-imidazolyl, 4-imidazolyl, 5-imidazolyl, 3-pyrazolyl, 4-pyrazolyl, isothiazolyl, isoxazolyl, 2-thiazolyl, 4-thiazolyl, 5-thiazolyl, 2-oxazolyl, 4-oxazolyl, 5-oxazolyl, 1,2,4-oxadiazol-5-yl, 1,2,4-triazol-3-yl, 1,2,3-triazol-4-yl, tetrazol-5-yl, benzimidazol-2-yl, indol-3-yl, 1H-indazol-3-yl, 1H-pyrrolo[2,3-b]pyrazin-2-yl, 1H-pyrrolo[2,3-b]pyridin-6-yl, 1H-imidazo[4,5-b]pyridin-2-yl, 1H-imidazo[4,5-c]pyridin-2-yl, 1H-imidazo[4,5-b]pyrazin-2-yl, benzopyranyl, dihydrobenzopyranyl and the like. The heterocyclic group is preferably a pyridyl, oxazolyl or thiazolyl group.

The hydrocarbon group and heterocyclic group represented by R in the formula [I] optionally have 1 to 5, preferably 1 to 3, substituents at any substitutable position. Examples of the substituent include an aliphatic hydrocarbon group, an alicyclic hydrocarbon group, an aryl group, an aromatic heterocyclic group, a non-aromatic heterocyclic group, a halogen atom, a nitro group, an optionally substituted amino group, an optionally substituted acyl group, an optionally substituted hydroxyl group, an optionally substituted thiol group, an optionally esterified carboxyl group, an amidino group, a carbamoyl group, a sulfamoyl group, a sulfo group, a cyano group, an azide group and a nitroso group.

Examples of the aliphatic hydrocarbon group include a straight-chain or branched aliphatic hydrocarbon group having 1 to 15 carbon atoms, such as alkyl group, alkenyl group, alkynyl group and the like.

Preferable examples of the alkyl group include an alkyl group having 1 to 10 carbon atoms, such as methyl, ethyl, propyl, isopropyl, butyl, isobutyl, sec.-butyl, t.-butyl, pentyl, isopentyl, neopentyl, t.-pentyl, 1-ethylpropyl, hexyl, isohexyl, 1,1-dimethylbutyl, 2,2-dimethylbutyl, 3,3-dimethylbutyl, 2-ethylbutyl, hexyl, pentyl, octyl, nonyl, decyl and the like.

Preferable examples of the alkenyl group include an alkenyl group having 2 to 10 carbon atoms, such as vinyl, allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like.

Preferable examples of the alkynyl group include an alkynyl group having 2 to 10 carbon atoms, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like.

As the alicyclic hydrocarbon group, a saturated or unsaturated alicyclic hydrocarbon group having 3 to 12 carbon atoms, such as cycloalkyl group, cycloalkenyl group, cycloalkadienyl group and the like, is exemplified.

Preferable examples of the cycloalkyl group include a cycloalkyl group having 3 to 10 carbon atoms, such as cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl and the like.

Preferable examples of the cycloalkenyl group include a cycloalkenyl group having 3 to 10 carbon atoms, such as 2-cyclopenten-1-yl, 3-cyclopenten-1-yl, 2-cyclohexen-1-yl, 3-cyclohexen-1-yl and the like.

Preferable examples of the cycloalkadienyl group include a cycloalkadienyl group having 4 to 10 carbon atoms, such as 2,4-cyclopentadien-1-yl, 2,4-cyclohexadien-1-yl, 2,5-cyclohexadien-1-yl and the like.

Preferable examples of the aryl group include an aryl group having 6 to 14 carbon atoms, such as phenyl, naphthyl (1-naphthyl, 2-naphthyl), anthryl, phenanthryl, acenaphthylenyl and the like.

Preferable examples of the aromatic heterocyclic group include an aromatic monocyclic heterocyclic group such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like; an aromatic fused heterocyclic group such as benzofuranyl, isobenzofuranyl, benzo[b]thienyl, indolyl, isoindolyl, 1H-indazolyl, benzimidazolyl, benzoxazolyl, 1,2-benzisoxazolyl, benzothiazolyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl and the like; and the like.

Preferable examples of the non-aromatic heterocyclic group include oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl, pyrrolidino, piperidino, morpholino, thiomorpholino and the like.

Examples of the halogen atom include fluorine, chlorine, bromine and iodine.

In the optionally substituted amino group, the substituted amino group is exemplified by an N-monosubstituted amino group and an N,N-disubstituted amino group. Examples of the substituted amino group include an amino group having 1 or 2 substituents from among C₁₋₁₀ alkyl group, C₂₋₁₀ alkenyl group, C₂₋₁₀ alkynyl group, aromatic group, heterocyclic group and C₁₋₁₀ acyl group, which is exemplified by methylamino, dimethylamino, ethylamino, diethylamino, dibutylamino, diallylamino, cyclohexylamino, phenylamino, N-methyl-N-phenylamino, acetylamino, propionylamino, benzoylamino, nicotinoylamino and the like.

Examples of the acyl group of the optionally substituted acyl group include an acyl group having 1 to 13 carbon atoms, such as an alkanoyl group having 1 to 10 carbon atoms, an alkenoyl group having 3 to 10 carbon atoms, a cycloalkanoyl group having 4 to 10 carbon atoms, a cycloalkenoyl group having 4 to 10 carbon atoms, an aromatic carbonyl group having 6 to 12 carbon atoms and the like.

Preferable examples of the alkanoyl group having 1 to 10 carbon atoms include formyl, acetyl, propionyl, butyryl, isobutyryl, valeryl, isovaleryl, pivaloyl, hexanoyl, heptanoyl, octanoyl and the like.

Preferable examples of the alkenoyl group having 3 to 10 carbon atoms include acryloyl, methacryloyl, crotonoyl, isocrotonoyl and the like.

Preferable examples of the cycloalkanoyl group having 4 to 10 carbon atoms include cyclobutanecarbonyl, cyclopentanecarbonyl, cyclohexanecarbonyl, cycloheptanecarbonyl and the like.

Preferable examples of the cycloalkenoyl group having 4 to 10 carbon atoms include 2-cyclohexenecarbonyl and the like.

Preferable examples of the aromatic carbonyl group having 6 to 12 carbon atoms include benzoyl, naphthoyl, nicotinoyl and the like.

The substituent for the substituted acyl group includes, for example, an alkyl group having 1 to 3 carbon atoms, an alkoxy group having 1 to 3 carbon atoms, a halogen atom (e.g., chlorine, fluorine, bromine etc.), a nitro group, a hydroxyl group, an amino group and the like.

In the optionally substituted hydroxyl group, the substituted hydroxyl group is exemplified by an alkoxy group, a cycloalkyloxy group, an alkenyloxy group, a cycloalkenyloxy group, an aralkyloxy group, an acyloxy group, an aryloxy group and the like.

Preferable examples of the alkoxy group include an alkoxy group having 1 to 10 carbon atoms, such as methoxy, ethoxy, propoxy, isopropoxy, butoxy, isobutoxy, sec.-butoxy, t.-butoxy, pentyloxy, isopentyloxy, neopentyloxy, hexyloxy, heptyloxy, nonyloxy and the like.

Preferable examples of the cycloalkyloxy group include a cycloalkyloxy group having 3 to 10 carbon atoms, such as cyclobutoxy, cyclopentyloxy, cyclohexyloxy and the like.

Preferable examples of the alkenyloxy group include an alkenyloxy group having 2 to 10 carbon atoms, such as allyloxy, crotyloxy, 2-pentenyloxy, 3-hexenyloxy and the like.

Preferable examples of the cycloalkenyloxy group include a cycloalkenyloxy group having 3 to 10 carbon atoms, such as 2-cyclopentenyloxy, 2-cyclohexenyloxy and the like.

Preferable examples of the aralkyloxy group include an aralkyloxy group having 7 to 10 carbon atoms, such as phenyl-C₁₋₄ alkyloxy (e.g., benzyloxy, phenethyloxy etc.) and the like.

Preferable examples of the acyloxy group include an acyloxy group having 2 to 13 carbon atoms, more preferably an alkanoyloxy group having 2 to 4 carbon atoms (e.g., acetyloxy, propionyloxy, butyryloxy, isobutyryloxy etc.) and the like.

Preferable examples of the aryloxy group include an aryloxy group having 6 to 14 carbon atoms, such as phenoxy, naphthyloxy and the like. The aryloxy group may have 1 or 2 substituents. Examples of such substituent include a halogen atom (e.g., chlorine, fluorine, bromine etc.) and the like. Examples of the substituted aryloxy group include 4-chlorophenoxy and the like.

In the optionally substituted thiol group, the substituted thiol group is exemplified by an alkylthio group, a cycloalkylthio group, an alkenylthio group, a cycloalkenylthio group, an aralkylthio group, an acylthio group, an arylthio group and the like.

Preferable examples of the alkylthio group include an alkylthio group having 1 to 10 carbon atoms, such as methylthio, ethylthio, propylthio, isopropylthio, butylthio, isobutylthio, sec.-butylthio, t.-butylthio, pentylthio, isopentylthio, neopentylthio, hexylthio, heptylthio, nonylthio and the like.

Preferable examples of the cycloalkylthio group include a cycloalkylthio group having 3 to 10 carbon atoms, such as cyclobutylthio, cyclopentylthio, cyclohexylthio and the like.

Preferable examples of the alkenylthio group include an alkenylthio group having 2 to 10 carbon atoms, such as allylthio, crotylthio, 2-pentenylthio, 3-hexenylthio and the like.

Preferable examples of the cycloalkenylthio group include a cycloalkenylthio group having 3 to 10 carbon atoms, such as 2-cyclopentenylthio, 2-cyclohexenylthio and the like.

Preferable examples of the aralkylthio group include an aralkylthio group having 7 to 10 carbon atoms, such as phenyl-C₁₋₄ alkylthio (e.g., benzylthio, phenethylthio etc.) and the like.

Preferable examples of the acylthio group include an acylthio group having 2 to 13 carbon atoms, more preferably an alkanoylthio group having 2 to 4 carbon atoms (e.g., acetylthio, propionylthio, butyrylthio, isobutyrylthio and the like) and the like.

Preferable examples of the arylthio group include an arylthio group having 6 to 14 carbon atoms, such as phenylthio, naphthylthio and the like. The arylthio group may have 1 or 2 substituents. Examples of such substituent include a halogen atom (e.g., chlorine, fluorine, bromine etc.) and the like. Examples of the substituted arylthio group include 4-chlorophenylthio and the like.

Examples of the optionally esterified carboxyl group include an alkoxycarbonyl group, an aralkyloxycarbonyl group, an aryloxycarbonyl group and the like.

Preferable examples of the alkoxycarbonyl group include an alkoxycarbonyl group having 2 to 5 carbon atoms, such as methoxycarbonyl, ethoxycarbonyl, propoxycarbonyl, butoxycarbonyl and the like.

Preferable examples of the aralkyloxycarbonyl group include an aralkyloxycarbonyl group having 8 to 10 carbon atoms, such as benzyloxycarbonyl and the like.

Preferable examples of the aryloxycarbonyl group include an aryloxycarbonyl group having 7 to 15 carbon atoms, such as phenoxycarbonyl, p-tolyloxycarbonyl and the like.

The substituent for the hydrocarbon group and heterocyclic group represented by R is preferably an alkyl group having 1 to 10 carbon atoms, an aromatic heterocyclic group or an aryl group having 6 to 14 carbon atoms, more preferably C₁₋₃ alkyl, furyl, thienyl, phenyl and naphthyl.

When the substituent(s) on the hydrocarbon group and heterocyclic group represented by R in the formula [I] is(are) an alicyclic hydrocarbon group, an aryl group, an aromatic heterocyclic group or a non-aromatic heterocyclic group, the substituent(s) may further have one or more, preferably 1 to 3, suitable substituents. Examples of such substituent include an alkyl group having 1 to 6 carbon atoms, an alkenyl group having 2 to 6 carbon atoms, an alkynyl group having 2 to 6 carbon atoms, a cycloalkyl group having 3 to 7 carbon atoms, an aryl group having 6 to 14 carbon atoms, an aromatic heterocyclic group (e.g., thienyl, furyl, pyridyl, oxazolyl, thiazolyl etc.), a non-aromatic heterocyclic group (e.g., tetrahydrofuryl, morpholino, thiomorpholino, piperidino, pyrrolidino, piperazino etc.), an aralkyl group having 7 to 9 carbon atoms, an amino group, an N-mono-C₁₋₄ alkylamino group, an N,N-di-C₁₋₄ alkylamino group, an acylamino group having 2 to 8 carbon atoms (e.g., acetylamino, propionylamino, benzoylamino etc.), an amidino group, an acyl group having 2 to 8 carbon atoms (e.g., alkanoyl group having 2 to 8 carbon atoms etc.), a carbamoyl group, an N-mono-C₁₋₄ alkylcarbamoyl group, an N,N-di-C₁₋₄ alkylcarbamoyl group, a sulfamoyl group, an N-mono-C₁₋₄ alkylsulfamoyl group, an N,N-di-C₁₋₄ alkylsulfamoyl group, a carboxyl group, an alkoxycarbonyl group having 2 to 8 carbon atoms, a hydroxyl group, an alkoxy group having 1 to 4 carbon atoms, an alkenyloxy group having 2 to 5 carbon atoms, a cycloalkyloxy group having 3 to 7 carbon atoms, an aralkyloxy group having 7 to 9 carbon atoms, an aryloxy group having 6 to 14 carbon atoms, a mercapto group, an alkylthio group having 1 to 4 carbon atoms, an aralkylthio group having 7 to 9 carbon atoms, an arylthio group having 6 to 14 carbon atoms, a sulfo group, a cyano group, an azido group, a nitro group, a nitroso group, a halogen atom and the like.

In the formula [I], R is preferably an optionally substituted heterocyclic group. More preferably, R is a pyridyl, oxazolyl or thiazolyl group optionally having 1 to 3 substituent(s) selected from C₁₋₃ alkyl, furyl, thienyl, phenyl and naphthyl.

In the formula [I], Y is -CO-, -CH(OH)- or -NR³- (wherein R³ is an optionally substituted alkyl group), with preference given to -CH(OH)- and -NR³-. As used herein, the alkyl group of the optionally substituted alkyl group represented by R³ is exemplified by an alkyl having 1 to 4 carbon atoms, such as methyl, ethyl, propyl, isopropyl, butyl, isobutyl, sec.-butyl, t.-butyl and the like. As the substituent, for example, a halogen atom (e.g., fluorine, chlorine, bromine, iodine), an alkoxy group having 1 to 4 carbon atoms (e.g., methoxy, ethoxy, propoxy, butoxy, isobutoxy, sec.-butoxy, t.-butoxy etc.), a hydroxyl group, a nitro group, an acyl group having 1 to 4 carbon atoms (e.g., formyl, acetyl, propionyl etc.) and the like are mentioned.
The "m" is 0 or 1, preferably 0.
The "n" is 0, 1 or 2, preferably 0 or 1.
The "X" is CH or N, preferably CH.

In the formula [I], A is a bond or a divalent aliphatic hydrocarbon group having 1 to 7 carbon atoms. The aliphatic hydrocarbon group may be straight-chain or branched and saturated or unsaturated. Specific examples thereof include saturated ones such as -CH₂-, -CH(CH₃)-, -(CH₂)₂-, -CH(C₂H₅)-, -(CH₂)₃-, -(CH₂)₄-, -(CH₂)₅-, -(CH₂)₆-, -(CH₂)₇- and the like, and unsaturated ones such as -CH=CH-, -C(CH₃)=CH-, -CH=CH-CH₂-, -C(C₂H₅)=CH-, -CH₂-CH=CH-CH₂-, -CH₂-CH₂-CH=CH-CH₂-, -CH=CH-CH=CH-CH₂-, -CH=CH-CH=CH-CH=CH-CH₂- and the like. A is preferably a bond or a divalent aliphatic hydrocarbon group having 1 to 4 carbon atoms, wherein the aliphatic hydrocarbon group is preferably saturated. A is more preferably a bond or -(CH₂)₂-.

As the alkyl group represented by R¹, those exemplified for the aforementioned alkyl group represented by R³ are used. R¹ is preferably a hydrogen atom.

In the formula [I], the partial structural formula 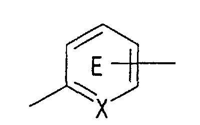 preferably represents the formula 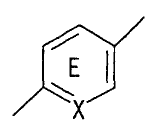 wherein each symbol is as defined above.

The ring E may further have 1 to 4 substituents at any substitutable position. Examples of such substituent include an alkyl group, an optionally substituted hydroxyl group, a halogen atom, an optionally substituted acyl group, a nitro group and an optionally substituted amino group. As these, those mentioned for the substituent of the aforementioned hydrocarbon group and heterocyclic group represented by R can be used.

The ring E, namely, the partial structural formula 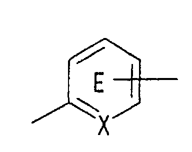 preferably represents the formula 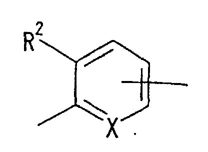 wherein R² is a hydrogen atom, an alkyl group, an optionally substituted hydroxyl group, a halogen atom, an optionally substituted acyl group, a nitro group or an optionally substituted amino group.

As the alkyl group, the optionally substituted hydroxyl group, the halogen atom, the optionally substituted acyl group and the optionally substituted amino group represented by R²_{,} those mentioned for the substituent of the aforementioned hydrocarbon group and heterocyclic group represented by R can be used. R² is preferably a hydrogen atom, an optionally substituted hydroxyl group or a halogen atom. R² is more preferably a hydrogen atom or an optionally substituted hydroxyl group, particularly preferably a hydrogen atom or an alkoxy group having 1 to 4 carbon atoms.

In the formula [I], L and M are each a hydrogen atom or show a bond in combination, with preference given to a hydrogen atom.

A compound wherein L and M are bonded to each other to form a bond has an (E) isomer and a (Z) isomer with regard to the double bond at the 5-position of the azolidinedione ring.

A compound wherein L and M are each a hydrogen atom has optical isomers of an (R)-form and an (S)-form with regard to the asymmetric carbon at the 5-position of the azolidinedione ring, and the compound encompasses optically active forms and racemates of these (R)-form and (S)-form.

Preferable examples of the compound of the formula [I] include a compound wherein R is a pyridyl, oxazolyl or thiazolyl group optionally having 1 to 3 substituent(s) selected from C₁₋₃ alkyl, furyl, thienyl, phenyl and naphthyl; m is 0; n is 0 or 1; X is CH; A is a bond or -(CH₂)₂-; R¹ is a hydrogen atom; ring E, namely the partial structural formula 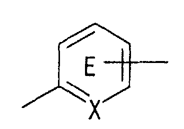 is the formula 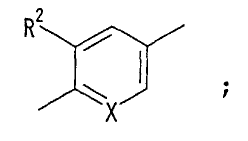 R² is a hydrogen atom or a C₁₋₄ alkoxy group; and L and M are each a hydrogen atom.

Preferable examples of the compound represented by the formula [I] include
5-[4-[2-(5-ethyl-2-pyridyl)ethoxy]benzyl]-2,4-thiazolidinedione (generic name: pioglitazone);
5-[[4-[(3,4-dihydro-6-hydroxy-2,5,7,8-tetramethyl-2H-1-benzopyran-2-yl)methoxy]phenyl]methyl]-2,4-thiazolidinedione (generic name: troglitazone);
5-[[4-[2-(methyl-2-pyridinylamino)ethoxy]phenyl]methyl]-2,4-thiazolidinedione (generic name: rosiglitazone);
5-[3-[4-(5-methyl-2-phenyl-4-thiazolylmethoxy)phenyl]propyl]-2,4-oxazolidinedione and the like.

The salt of the compound of the formula [I] includes a pharmacologically acceptable salt, such as a salt with an inorganic base, a salt with an organic base, a salt with an inorganic acid, a salt with an organic acid, a salt with an basic or acidic amino acid and the like.

Preferable examples of the salts with inorganic base include salts with alkali metals such as sodium, potassium and the like, alkaline earth metals such as calcium, magnesium and the like, and aluminum, ammonium and the like.

Preferable examples of the salts with organic base include salts with trimethylamine, triethylamine, pyridine, picoline, ethanolamine, diethanolamine, triethanolamine, dicyclohexylamine, N,N-dibenzylethylenediamine and the like.

Preferable examples of the salts with inorganic acid include salts with hydrochloric acid, hydrobromic acid, nitric acid, sulfuric acid, phosphoric acid and the like.

Preferable examples of the salts with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salts with basic amino acid include salts with arginine, lysin, ornithine and the like, and preferable examples of the salts with acidic amino acid include salts with aspartic acid, glutamic acid and the like.

The compound of the formula [I] or a salt thereof is preferably pioglitazone, rosiglitazone or salts thereof, more preferably pioglitazone or a hydrochloride thereof, rosiglitazone or a maleate thereof, particularly preferably pioglitazone hydrochloride.

The compound of the formula [I] or a salt thereof can be. produced according to the method described in, for example, JP-A-55-22636 (EP-A 8203), JP-A-60-208980 (EP-A 155845), JP-A-61-286376 (EP-A 208420), JP-A-61-85372 (EP-A 177353), JP-A-61-267580 (EP-A 193256), JP-A-5-86057 (WO 92/18501), JP-A-7-82269 (EP-A 605228), JP-A-7-101945 (EP-A 612743), EP-A-643050, EP-A-710659 and the like or a method analogous thereto.

The insulin sensitizer to be used in the present invention includes, besides the above-mentioned,
(±)-4-[4-[2-(5-methyl-2-phenyloxazol-4-yl)ethoxy]benzyl]-isoxazolidine-3,5-dione (JTT-501) or a salt thereof;
5-[[3,4-dihydro-2-(phenylmethyl)-2H-1-benzopyran-6-yl]methyl]-2,4-thiazolidinedione (generic name: englitazone) or a salt thereof (preferably sodium salt);
5-[[4-[3-(5-methyl-2-phenyl-4-oxazolyl)-1-oxopropyl]phenyl]methyl]-2,4-thiazolidinedione (generic name: darglitazone/CP-86325) or a salt thereof (preferably sodium salt);
5-[2-(5-methyl-2-phenyl-4-oxazolylmethyl)benzofuran-5-ylmethyl]-2,4-oxazolidinedione (CP-92768) or a salt thereof;
5-(2-naphthalenylsulfonyl)-2,4-thiazolidinedione (AY-31637) or a salt thereof;
4-[(2-naphthalenyl)methyl]-3H-1,2,3,5-oxathiadiazole-2-oxide (AY-30711) or a salt thereof;
5-[[6-(2-fluorobenzyloxy)-2-naphthyl]methyl]-2,4-thiazolidinedione (MCC-555) or a salt thereof; (±)-[5-[(2,4-dioxothiazolidin-5-yl)methyl]-2-methoxy-N-[[4-(trifluoromethyl)phenyl]methyl]benzamide (AHG-255) or a salt thereof;
4-[1-(3,5,5,8,8-pentamethyl-5,6,7,8-tetrahydronaphthalen-2-yl)ethenyl]benzoic acid (LGD1069) or a salt thereof;
6-[1-(3,5,5,8,8-pentamethyl-5,6,7,8-tetrahydronaphthalen-2-yl)cyclopropyl]nicotinic acid (LG100268) or a salt thereof;
1,4-bis[4-[(3,5-dioxo-1,2,4-oxadiazolidin-2-yl)methyl]phenoxy]-2-butene (YM-440) or a salt thereof;
CS-011; dexlipotam; GI-262570; INS-1; AR-H-0329242; CLX-0901; FK-614; KRP-297; CRE-16336; NN-2344; BM-13-1258; S-15261; KB-R-7785; DN-108; DRF-2725; GW-2570; GW-2433; MXC-3255; L-746449; L-767827; L-783281 and the like.

As the salts of these compounds, those mentioned for the aforementioned salt of the compound of the formula [I] can be used.

The insulin sensitizer is preferably pioglitazone or a hydrochloride thereof, rosiglitazone or a maleate thereof, or (±)-4-[4-[2-(5-methyl-2-phenyloxazol-4-yl)ethoxy]benzyl]isoxazolidine-3,5-dione, particularly preferably pioglitazone hydrochloride.

The insulin sensitizer to be used in the present invention may be a mixture of two or more kinds thereof mixed at a suitable ratio.

The HMG-CoA reductase inhibitor to be used in the present invention is a pharmaceutical agent that inhibits 3-hydroxy-3-methylglutaryl-coenzyme A reductase (HMG-CoA reductase), which is a rate-determining enzyme in cholesterol biosynthesis, and is exemplified by a statin compound and the like. Specific examples thereof include cerivastatin, pravastatin, simvastatin, lovastatin, atorvastatin, fluvastatin, itavastatin, ZD-4522 or salts thereof and the like.

As the salt here, those mentioned for the salt of the aforementioned compound of the formula [I] can be used.

The HMG-CoA reductase inhibitor is preferably cerivastatin, pravastatin, atorvastatin or salts thereof and the like.

The HMG-CoA reductase inhibitor to be used in the present invention may be a mixture of two or more kinds thereof mixed at a suitable ratio.

Examples of preferable combination for the agent of the present invention include
1) a combination of pioglitazone or a salt thereof (preferably hydrochloride) and cerivastatin or a salt thereof (preferably sodium salt);
2) a combination of pioglitazone or a salt thereof (preferably hydrochloride) and pravastatin or a salt thereof (preferably sodium salt);
3) a combination of pioglitazone or a salt thereof (preferably hydrochloride) and atorvastatin or a salt thereof;
4) a combination of rosiglitazone or a salt thereof (preferably maleate) and cerivastatin or a salt thereof (preferably sodium salt);
5) a combination of rosiglitazone or a salt thereof (preferably maleate) and pravastatin or a salt thereof (preferably sodium salt);
6) a combination of rosiglitazone or a salt thereof (preferably maleate) and atorvastatin or a salt thereof, and the like.

In the present invention, the TNF-α inhibitor means a pharmaceutical agent that decreases the production amount of TNF-α or TNF-α activity in biological tissues (e.g., skeletal muscle, monocyte, macrophage, neutrophile, fibroblast, epithelial cell, astrocyte etc.).

The TNF-α inhibitor of the present invention is used as an agent for the prophylaxis or treatment of a disease in which TNF-α is involved (disease induced by TNF-α) in a mammal (e.g., human, mouse, rat, rabbit, dog, cat, cattle, horse, pig, monkey etc.). As used herein, the disease in which TNF-α is involved means a disease developed by the presence of TNF-α and treated via a TNF-α inhibitory effect. Examples of such disease include inflammatory diseases such as diabetic complications (e.g., retinopathy, nephropathy, neuropathy, macroangiopathy etc.; arthritis (e.g., rheumatoid arthritis, osteoarthritis, rheumatoid myelitis, gouty arthritis, periostitis etc.; lumbago; gout; post-operative/post-traumatic inflammation; remission of swelling; neuralgia; pharyngitis; cystitis; pneumonia; atopic dermatitis; inflammatory bowel diseases (e.g., Crohn's disease, ulcerative colitis etc.; meningitis; inflammatory ocular disease; and inflammatory pulmonary diseases (e.g., pneumonia, pulmonary silicosis, pulmonary sarcoidosis, pulmonary tuberculosis etc.), cardiovascular diseases (e.g., angina pectoris, cardiac infarction, congestive heart failure, disseminated intravascular coagulation etc.), asthma, allergic disease, chronic obstructive pulmonary disease, systemic lupus erythematosus, Crohn's disease, autoimmune hemolytic anemia, psoriasis, neurodegenerative diseases (e.g., Alzheimer's disease, Parkinson's disease, amyotrophic lateral sclerosis, AIDS encephalopathy etc.), central nervous system disorder (e.g., cerebrovascular disorders such as cerebral hemorrhage and cerebral infarction etc., head injury, spinal injury, cerebral edema, multiple sclerosis etc.), toxemia (e.g., sepsis, septic shock, endotoxin shock, gram negative sepsis, toxic shock syndrome etc.), Addison's disease, Creutzfeldt-Jakob disease, viral infectious disease (e.g., viral infectious diseases such as cytomegalovirus, influenza virus, herpesvirus etc.), rejection by transplantation, dialysis hypotension, osteoporosis and the like.

The agent of the present invention can be obtained by combining an insulin sensitizer and an HMG-CoA reductase inhibitor, that are the active ingredients. These active ingredients may be formulated by mixing separately or simultaneously with a pharmacologically acceptable carrier according to a method known *per se* [conventional method in the technical field of manufacturing pharmaceutical preparations, such as the method described in the Japanese Pharmacopoeia (e.g., JP XIII)].

The dosage form of the agent of the present invention or each active ingredient thereof is, for example, an oral preparation such as tablet, capsule (including soft capsule and microcapsule), powder, granule, syrup and the like; and a parenteral preparation such as injection (e.g., subcutaneous injection, intravenous injection, intramuscular injection, intraperitoneal injection etc.), external preparation (e.g., preparation for nasal administration, percutaneous preparation, ointment etc.), suppository (e.g., rectal suppository, vaginal suppository etc.), pellet, drops, sustained-release preparation (e.g., sustained-release microcapsule etc.) and the like.

The production methods of oral preparations and parenteral preparations are explained in detail in the following.

An oral preparation can be produced by adding, to the active ingredients, for example, an excipient (e.g., lactose, sucrose, starch, D-mannitol, xylitol, sorbitol, erythritol, crystalline cellulose, light silicic anhydride etc.), a disintegrant (e.g., calcium carbonate, starch, carboxymethyl cellulose, carboxymethyl cellulose calcium, low-substituted hydroxypropylcellulose, croscarmellose sodium, carboxymethyl starch sodium, light silicic anhydride etc.), a binder (e.g., pregelatinized starch, gum arabic, carboxymethyl cellulose, hydroxypropylcellulose, hydroxypropylmethylcellulose, polyvinylpyrrolidone, crystalline cellulose, methyl cellulose, sucrose, D-mannitol, trehalose, dextrin etc.), a lubricant (e.g., talc, magnesium stearate, calcium stearate, colloidal silica, polyethylene glycol 6000 etc.) and the like, and compression-molding the mixture. To the oral preparation, an acid such as hydrochloric acid, phosphoric acid, malonic acid, succinic acid, DL-malic acid, tartaric acid, maleic acid, fumaric acid, citric acid and the like or a base such as sodium carbonate, sodium hydrogencarbonate, sodium citrate, sodium tartrate and the like may be added for promoting dissolution of the active ingredients.

Furthermore, the oral preparation may be coated by a method known per se, for the purpose of masking a taste, enteric coating, or achieving a sustained release. Examples of the coating agent include an enteric polymer (e.g., cellulose acetate phthalate, methacrylate copolymer L, methacrylic acid copolymer LD, methacrylic acid copolymer S, hydroxypropylmethylcellulose phthalate, hydroxypropylmethylcellulose acetate succinate, carboxymethylethylcellulose etc.), a gastric coating polymer (e.g., polyvinylacetal diethylaminoacetate, aminoalkyl methacrylate copolymer E etc.), a water-soluble polymer (e.g., hydroxypropylcellulose, hydroxypropylmethylcellulose etc.), a water-insoluble polymer (e.g., ethylcellulose, aminoalkyl methacrylate copolymer RS, ethyl acrylate/methyl methacrylate copolymer etc.), wax and the like. For coating, a plasticizer such as polyethylene glycol etc. and a shading agent such as titanium oxide, iron sesquioxide etc. may be used, along with the above-mentioned coating agents.

An injection can be produced by dissolving, dispersing or emulsifying an active ingredient in an aqueous solvent (e.g., distilled water, physiological brine, Ringer's solution etc.), an oily solvent (e.g., vegetable oil such as olive oil, sesame oil, cottonseed oil, corn oil etc.; propylene glycol, macrogol, tricaprylin etc.) and the like, together with a dispersant [e.g., Tween 80 (Atlas Powder Co., U.S.A.), HCO 60 (Nikko Chemicals Co., Ltd.), polyethylene glycol, carboxymethyl cellulose, sodium alginate etc.], a preservative (e.g., methyl paraben, propyl paraben, benzyl alcohol, chlorobutanol, phenol etc.), an isotonizing agent (e.g., sodium chloride, glycerine, D-sorbitol, D-mannitol, xylitol, glucose, fructose etc.) and the like.

Where desired, additives may be used, such as a dissolution aid (e.g., sodium salicylate, sodium acetate, polyethylene glycol, propylene glycol, D-mannitol, trehalose, benzyl benzoate, ethanol, Tris aminomethane, cholesterol, triethanolamine, sodium carbonate, sodium citrate etc.), a suspending agent (e.g., surfactant such as stearyltriethanolamine, sodium lauryl sulfate, laurylaminopropionic acid, lecithin, benzalkonium chloride, benzethonium chloride, glycerine monostearate and the like); a hydrophilic polymer such as polyvinyl alcohol, polyvinylpyrrolidone, carboxymethylcellulose sodium, methylcellulose, hydroxymethylcellulose, hydroxyethylcellulose, hydroxypropylcellulose etc.), a buffering agent (e.g., buffer of phosphate, acetate, carbonate, citrate etc.), a stabilizer (e.g., human serum albumin etc.), a soothing agent (e.g., propylene glycol, lidocaine hydrochloride, benzyl alcohol etc.), a preservative (e.g., p-oxybenzoic acid esters, chlorobutanol, benzalkonium chloride, benzyl alcohol, phenethyl alcohol, dehydroacetic acid, sorbic acid etc.), and the like.

An external preparation can be produced by forming an active ingredient into a solid, semisolid or liquid composition. For example, the above-mentioned solid composition can be produced by making a powder of an active ingredient as it is or upon addition of and mixing with an excipient (e.g., lactose, D-mannitol, starch, crystalline cellulose, sucrose and the like), a thickening agent (e.g., natural rubbers, cellulose derivative, acrylate polymer etc.) and the like. The above-mentioned liquid composition can be produced in almost the same manner as in the case of injections. A semisolid composition is preferably an aqueous or oily gel, or an ointment. These compositions may contain a pH adjuster (e.g., phosphoric acid, citric acid, hydrochloric acid, sodium hydroxide etc.), a preservative (e.g., p-oxybenzoic acid esters, chlorobutanol, benzalkonium chloride, benzyl alcohol, phenethyl alcohol, dehydroacetic acid, sorbic acid etc.), and the like.

A suppository is produced by forming an active ingredient into an oily or aqueous solid, semisolid or liquid composition. As an oily base to be used for production of the composition may be, for example, higher fatty acid glyceride [e.g., cocoa butter, witepsols (Huels Aktiengesellschaft, Germany) etc.], a medium chain fatty acid triglyceride [e.g., Migriols (Huels Aktiengesellschaft, Germany) etc.], a vegetable oil (e.g., sesame oil, soybean oil, cottonseed oil etc.), and the like. As an aqueous base, for example, polyethylene glycols, propylene glycol and the like are mentioned. As an aqueous gel base, for example, natural rubber, cellulose derivative, vinyl polymer, acrylate polymer and the like are mentioned.

The mode of administration of the agent of the present invention is not particularly limited, as long as an insulin sensitizer and an HMG-CoA reductase inhibitor are combined on administration. Examples of such administration mode include (1) administration of a single preparation obtained by simultaneous formulation of the insulin sensitizer and the HMG-CoA reductase inhibitor, (2) simultaneous administration of two kinds of preparations obtained by separate preparation of the insulin sensitizer and the HMG-CoA reductase inhibitor, by the same administration route, (3) time stagger administration of two kinds of preparations obtained by separate preparation of the insulin sensitizer and the HMG-CoA reductase inhibitor, by the same administration route, (4) simultaneous administration of two kinds of preparations obtained by separate preparation of the insulin sensitizer and the HMG-CoA reductase inhibitor, by different administration routes, (5) time stagger administration of two kinds of preparations obtained by separate preparation of the insulin sensitizer and the HMG-CoA reductase inhibitor, by different administration routes, such as administration in the order of the insulin sensitizer and then the HMG-CoA reductase inhibitor, or in a reversed order, and the like. Of these, the above-mentioned (2) and (3) are preferable.

More specifically, it is preferable that the insulin sensitizer and the HMG-CoA reductase inhibitor are separately formed into oral preparations such as tablet and the like, and that the oral preparations are administered simultaneously or in a time staggered manner.

The agent of the present invention shows low toxicity and can be administered safely to a mammal (e.g., human, mouse, rat, rabbit, dog, cat, cattle, horse, pig, monkey etc.) orally or parenterally.

The dose of the agent of the present invention follows the dose of each pharmaceutical agent and can be appropriately determined depending on the administration subject, age and body weight of the administration subject, symptom, administration time, dosage form, administration route, combination of pharmaceutical agents and the like.

The doses of the insulin sensitizer and the HMG-CoA reductase inhibitor follow clinical doses and can be appropriately determined based thereon.

For example, when an insulin sensitizer is administered to an adult patient (body weight 50 kg, suffering from, for example, an inflammatory disease), the daily dose is generally 0.01 - 1000 mg, preferably 0.1 - 500 mg, which dose can be administered once or several times a day in divided portions.

When pioglitazone hydrochloride is used as an insulin sensitizer, the daily dose of pioglitazone hydrochloride is generally 7.5 - 60 mg, preferably 15 - 45 mg. When troglitazone is used as an insulin sensitizer, the daily dose of troglitazone is generally 100 - 1000 mg, preferably 200 - 600 mg. When rosiglitazone (or maleate thereof) is used as an insulin sensitizer, the daily dose of rosiglitazone is generally 1 - 12 mg, preferably 2 - 8 mg.

When an HMG-CoA reductase inhibitor is administered to an adult patient (body weight 50 kg, suffering from, for example, an inflammatory disease), the daily dose is generally 0.01 - 100 mg, preferably 0.5 - 50 mg, which dose can be administered once or several times a day in divided portions.

When cerivastatin sodium is used as an HMG-CoA reductase inhibitor, the daily dose of cerivastatin sodium is generally 0.01 - 1 mg, preferably 0.05 - 0.5 mg. When pravastatin sodium is used as an HMG-CoA reductase inhibitor, the daily dose of pravastatin sodium is generally 1 - 100 mg, preferably 5 - 50 mg. When simvastatin is used as an HMG-CoA reductase inhibitor, the daily dose of simvastatin is generally 0.5 - 50 mg, preferably 1 - 20 mg. When fluvastatin sodium is used as an HMG-CoA reductase inhibitor, the daily dose of fluvastatin sodium is generally 5 - 200 mg, preferably 10 - 100 mg.

The mixing ratio of an insulin sensitizer and an HMG-CoA reductase inhibitor in the agent of the present invention can be appropriately determined depending on the administration subject, age and body weight of the administration subject, symptom, administration time, dosage form, administration route, combination of pharmaceutical agents and the like. For example, an HMG-CoA reductase inhibitor is generally used in an amount of about 0.005 - 200 parts by weight, preferably about 0.01 - 0.2 part by weight, per part by weight of the insulin sensitizer.

The agent of the present invention shows an enhanced TNF-α inhibitory activity as compared to a single administration of an insulin sensitizer or an HMG-CoA reductase inhibitor.

Moreover, a combined use of an insulin sensitizer and an HMG-CoA reductase inhibitor affords reduction of the amount of the pharmaceutical agent to be used, as compared to the single use of respective pharmaceutical agents, which in turn reduces an unpreferable action of these pharmaceutical agents when they have such action.

In the agent of the present invention, a pharmaceutical agent for a combined use that does not adversely affect the insulin sensitizer or HMG-CoA reductase inhibitor can be used. Examples of such pharmaceutical agent for a combined use include "therapeutic agent of diabetes (excluding insulin sensitizers)", "therapeutic agent of diabetic complications", "anti-obesity agent", "therapeutic agent of hypertension", "therapeutic agent of hyperlipidemia (excluding HMG-CoA reductase inhibitors)", "diuretic agent" and the like.

The aforementioned "therapeutic agent of diabetes (excluding insulin sensitizers)" is exemplified by insulin, insulin secretagogues, biguanides, α-glucosidase inhibitors and the like.

As the insulin, any substance having an insulin activity can be used, which is exemplified by animal insulin extracted from the pancreas of cattle or pig; semi-synthetic human insulin enzymatically synthesized from insulin extracted from the pancreas of pig; human insulin genetically engineered *using E. coli* and yeast; and the like.

As the insulin, zinc insulin containing 0.45 - 0.9 (w/w)% of zinc; protamine zinc insulin produced from zinc chloride, protamine sulfate and insulin, and the like can be also used. The insulin may be a fragment or a derivative (e.g., INS-1 etc.) thereof.

The insulin includes various kinds such as ultra fast-acting type, fast-acting type, biphasic type, intermediate-acting type, long-acting type and the like. These can be selected as appropriate depending on the disease state of the patient.

When the agent of the present invention and insulin are to be combined for use, vascular complications and hypoglycemia induction are less likely to be caused, which are harmful effects of insulin overdose, because a synergistic effect can be afforded or the amount of insulin used is reduced as compared to that of the single administration thereof.

The insulin secretagogues include, for example, a sulfonylurea agent. Specific examples of the sulfonylurea agent include tolbutamide, chlorpropamide, tolazamide, acetohexamide, glyclopyramide and ammonium salt thereof, glibenclamide, gliclazide, 1-butyl-3-metanilylurea, carbutamide, glibornuride, glipizide, gliquidone, glisoxepide, glybuthiazole, glybuzole, glyhexamide, glymidine, glypinamide, phenbutamide, tolcyclamide, glimepiride and the like.

Besides the above-mentioned, the insulin secretagogues are exemplified by nateglinide (AY-4166), mitiglinide [calcium (2S)-2-benzyl-3-(cis-hexahydro-2-isoindolinylcarbonyl)propionate dihydrate (KAD-1229)], repaglinide and the like.

The biguanides are exemplified by phenformin, metformin, buformin and the like.

The α-glucosidase inhibitors are exemplified by acarbose, voglibose, miglitol, Emiglitate and the like.

Besides the above-mentioned, the "therapeutic agent of diabetes (excluding insulin sensitizers)" is exemplified by ergoset, pramlintide, leptin, BAY-27-9955, T-1095 and the like.

When, for example, a "therapeutic agent of diabetes (excluding insulin sensitizers)" is administered to an adult patient (body weight 50 kg), the daily dose is generally 0.1 - 2500 mg, preferably 0.5 - 1000 mg, which dose can be administered once or several times a day in divided portions.

When insulin is administered to an adult patient (body weight 50 kg, generally by injection), the daily dose is generally 10 - 100 U (unit), preferably 10 - 80 U (unit), which dose can be administered once or several times a day in divided portions.

When an insulin secretagogue is administered to an adult patient (body weight 50 kg), the daily dose is generally 0.1 - 1000 mg, preferably 1 - 100 mg, which dose can be administered once or several times a day in divided portions.

When a biguanide is administered to an adult patient (body weight 50 kg), the daily dose is generally 10 - 2500 mg, preferably 100 - 1000 mg, which dose can be administered once or several times a day in divided portions.

When an α-glucosidase inhibitor is administered to an adult patient (body weight 50 kg), the daily dose is generally 0.1 - 400 mg, preferably 0.6 - 300 mg, which dose can be administered once or several times a day in divided portions.

Examples of the aforementioned "therapeutic agent of diabetic complications" include an aldose reductase inhibitor, a glycation inhibitor, a protein kinase C inhibitor and the like.

Examples of the aldose reductase inhibitor include tolrestat; epalrestat; 3,4-dihydro-2,8-diisopropyl-3-thioxo-2H-1,4-benzoxazine-4-acetic acid; imirestat; zenarestat; 6-fluoro-2,3-dihydro-2',5'-dioxo-spiro[4H-1-benzopyran-4,4'-imidazolidine]-2-carboxamide (SNK-860); zopolrestat; sorbinil; and 1-[(3-bromo-2-benzofuranyl)sulfonyl]-2,4-imidazolidinedione (M-16209); CT-112; NZ-314; ARI-509 and the like.

Examples of the glycation inhibitor include pimagedine and the like.

Examples of the protein kinase C inhibitor include NGF, LY-333531 and the like.

Besides the above-mentioned, examples of the "therapeutic agent of diabetic complications" include alprostadil, tiapride hydrochloride, cilostazol, mexiletine hydrochloride, ethyl icosapentate, memantine, pimagedline (ALT-711) and the like.

When, for example, a "therapeutic agent of diabetic complications" is administered to an adult patient (body weight 50 kg), the daily dose is generally 0.1 - 2000 mg, which dose can be administered once or several times a day in divided portions.

When an aldose reductase inhibitor is administered to an adult patient (body weight 50 kg), the daily dose is generally 1 - 1000 mg, which dose can be administered once or several times a day in divided portions.

When a glycation inhibitor is administered to an adult patient (body weight 50 kg), the daily dose is generally 1 - 2000 mg, which dose can be administered once or several times a day in divided portions.

When a protein kinase C inhibitor is administered to an adult patient (body weight 50 kg), the daily dose is generally 0.1 - 100 mg, which dose can be administered once or several times a day in divided portions.

Examples of the aforementioned "anti-obesity agent" include a lipase inhibitor, an anorectic drug and the like.

Examples of the lipase inhibitor include orlistat and the like.

Examples of the anorectic drug include dexfenfluramine, fluoxetine, sibutramine, biamine and the like.

When, for example, an "anti-obesity agent" is administered to an adult patient (body weight 50 kg), the daily dose is generally 0.01 - 1000 mg, preferably 0.1 - 1000 mg, which dose can be administered once or several times a day in divided portions.

When a lipase inhibitor is administered to an adult patient (body weight 50 kg), the daily dose is generally 0.1 - 1000 mg, which dose can be administered once or several times a day in divided portions.

When an anorectic drug is administered to an adult patient (body weight 50 kg), the daily dose is generally 0.01 - 1000 mg, preferably 0.1 - 500 mg, which dose can be administered once or several times a day in divided portions.

Examples of the aforementioned "therapeutic agent of hypertension" include an angiotensin converting enzyme inhibitor, a calcium antagonist, a potassium channel opener and the like.

Examples of the angiotensin converting enzyme inhibitor include captopril, enalapril, alacepril, delapril, ramipril, lisinopril, imidapril, benazepril, ceronapril, cilazapril, enalaprilat, foshinopril, moveltopril, perindopril, quinapril, spirapril, temocapril, trandolapril, manidipine and the like.

Examples of the calcium antagonist include nifedipine, amlodipine, efonidipine, nicardipine and the like.

Examples of the potassium channel opener include levcromakalim, L-27152, AL 0671, NIP-121 and the like.

When, for example, a "therapeutic agent of hypertension" is administered to an adult patient (body weight 50 kg), the daily dose is generally 0.01 - 1000 mg, which dose can be administered once or several times a day in divided portions.

When an angiotensin converting enzyme inhibitor is administered to an adult patient (body weight 50 kg), the daily dose is generally 0.01 - 500 mg, preferably 0.1 - 100 mg, which dose can be administered once or several times a day in divided portions.

When a calcium antagonist is administered to an adult patient (body weight 50 kg), the daily dose is generally 0.1 - 500 mg, preferably 1 - 200 mg, which dose can be administered once or several times a day in divided portions.

When a potassium channel opener is administered to an adult patient (body weight 50 kg), the daily dose is generally 0.01 - 1000 mg, which dose can be administered once or several times a day in divided portions.

Examples of the aforementioned "therapeutic agent of hyperlipidemia (excluding HMG-CoA reductase inhibitors)" include a fibrate compound and the like.

Examples of the fibrate compound include bezafibrate, beclobrate, binifibrate, ciprofibrate, clinofibrate, clofibrate, clofibrinic acid, etofibrate, phenofibrate, gemfibrozil, nicofibrate, pirifibrate, ronifibrate, simfibrate, theofibrate and the like.

When, for example, a "therapeutic agent of hyperlipidemia (excluding HMG-CoA reductase inhibitors)" is administered to an adult patient (body weight 50 kg), the daily dose is generally 0.01 - 3000 mg, preferably 1 - 2000 mg, which dose can be administered once or several times a day in divided portions.

When a fibrate compound is administered to an adult patient (body weight 50 kg), the daily dose is generally 1 - 2000 mg, preferably 10 - 1500 mg, which dose can be administered once or several times a day in divided portions.

Examples of the aforementioned "diuretic agent" include a xanthine derivative preparation, a thiazide preparation, an antialdosterone preparation, a carbonic anhydrase inhibitor, a chlorobenzenesulfonamide preparation and the like.

Examples of the xanthine derivative preparation include theobromine sodium salicylate, theobromine calcium salicylate and the like.

Examples of the thiazide preparation include ethiazide, cyclopenthiazide, trichlormethiazide, hydrochlorothiazide, hydroflumethiazide, benzylhydrochlorothiazide, penflutizide, polythiazide, methyclothiazide and the like.

Examples of the antialdosterone preparation include spironolactone, triamterene and the like.

Examples of the carbonic anhydrase inhibitor include acetazolamide and the like.

Examples of the chlorobenzenesulfonamide preparation include chlorthalidone, mefruside, indapamide and the like.

Besides the above-mentioned, examples of the "diuretic agent" include azosemide, isosorbide, ethacrynic acid, piretanide, bumetanide, furosemide and the like.

When, for example, a "diuretic agent" is administered to an adult patient (body weight 50 kg), the daily dose is generally 0.01 mg - 100 g, preferably 0.05 mg - 10 g, which dose can be administered once or several times a day in divided portions.

When a xanthine derivative preparation is administered to an adult patient (body weight 50 kg), the daily dose is generally 0.1 - 100 g, preferably 0.5 - 10 g, which dose can be administered once or several times a day in divided portions.

When a thiazide preparation is administered to an adult patient (body weight 50 kg), the daily dose is generally 0.01 - 2000 mg, preferably 0.05 - 500 mg, which dose can be administered once or several times a day in divided portions.

When an antialdosterone preparation is administered to an adult patient (body weight 50 kg), the daily dose is generally 1 - 2000 mg, preferably 10 - 1000 mg, which dose can be administered once or several times a day in divided portions.

When a carbonic anhydrase inhibitor is administered to an adult patient (body weight 50 kg), the daily dose is generally 10 - 5000 mg, preferably 50 - 2000 mg, which dose can be administered once or several times a day in divided portions.

When a chlorobenzenesulfonamide preparation is administered to an adult patient (body weight 50 kg), the daily dose is generally 1 - 2000 mg, preferably 10 - 1000 mg, which dose can be administered once or several times a day in divided portions.

The aforementioned pharmaceutical agents for combined use may be used in combination of two or more optional agents thereof. Specific exemplary combinations when two kinds of pharmaceutical agents for combined use are to be used in combination include "a combination of an insulin secretagogue and a biguanide", "a combination of an insulin secretagogue and an α-glucosidase inhibitor", "a combination of insulin and a biguanide", "a combination of insulin and an α-glucosidase inhibitor" and the like.

The mode of administration of the agent of the present invention and a pharmaceutical agent for combined use is not particularly limited, as long as they are combined on administration.

The mixing ratio of the agent of the present invention and a pharmaceutical agent for combined use can be appropriately determined depending on the administration subject, age and body weight of the administration subject, symptom, administration time, dosage form, administration route and the like. For example, a pharmaceutical agent for combined use is used in an amount of 0.0001 - 10000 parts by weight per part by weight of the agent of the present invention.

The TNF-α inhibitory effect of the agent of the present invention can be evaluated by, for example, measuring the TNF-α amount in plasma using KKA^{y} mice, the genetically obesity and diabetes model.

That is, after an insulin sensitizer and an HMG-CoA reductase inhibitor are administered to a KKA^{y} mouse, which is a genetically obesity and diabetes model, the mouse is killed and the blood is taken. The obtained blood is separated by centrifugation and the TNF-α in plasma is quantitatively determined by an enzymatic immunoassay based on a biotin-streptavidin method.

### Best Mode for Embodying the Invention

The present invention is described in more detail by means of the Reference Examples and Example, which are not to be construed as limitative.

### Reference Example 1

Pioglitazone hydrochloride (2479.5 g, 2250 g as pioglitazone), lactose (13930.5 g) and carboxymethylcellulose calcium (carmellose calcium, 540 g) were placed in a fluidized-bed granulating and drying machine (manufactured by POWREX) and mixed with preheating. An aqueous solution (7500 g) containing hydroxypropylcellulose (450 g) dissolved therein was sprayed thereon to give granules. The obtained granules (16820 g) were passed through a cutter mill (manufactured by Showa Kagaku Kikai Kousakusho) to give milled powders. The obtained milled powders (16530 g), carmellose calcium (513 g) and magnesium stearate (57 g) were mixed in a tumbler mixer (manufactured by Showa Kagaku Kikai Kousakusho) and this mixture (16800 g) was tableted using a tableting machine (manufactured by Kikusui Seisakusho Ltd.) to give 140,000 tablets having the following composition, each tablet containing 15 mg of pioglitazone.

| Composition (unit: mg) per tablet: | |
|---|---|
| 1) pioglitazone hydrochloride | 16.53 |
| 2) lactose | 92.87 |
| 3) carmellose calcium | 7.2 |
| 4) hydroxypropylcellulose | 3.0 |
| 5) magnesium stearate | 0.4 |
| total | 120.0 |

### Reference Example 2

In the same manner as in Reference Example 1, 140,000 tablets having the following composition, each tablet containing 30 mg of pioglitazone, were obtained.

| Composition (unit: mg) per tablet: | |
|---|---|
| 1) pioglitazone hydrochloride | 33.06 |
| 2) lactose | 76.34 |
| 3) carmellose calcium | 7.2 |
| 4) hydroxypropylcellulose | 3.0 |
| 5) magnesium stearate | 0.4 |
| total | 120.0 |

### Reference Example 3

In the same manner as in Reference Example 2, 140,000 tablets having the following composition, each tablet containing 45 mg of pioglitazone, were obtained.

| Composition (unit: mg) per tablet: | |
|---|---|
| 1) pioglitazone hydrochloride | 49.59 |
| 2) lactose | 114.51 |
| 3) carmellose calcium | 10.8 |
| 4) hydroxypropylcellulose | 4.5 |
| 5) magnesium stearate | 0.6 |
| total | 180.0 |

### Example 1

After a pioglitazone hydrochloride 15 mg tablet and a pravastatin sodium 5 mg tablet are simultaneously administered to a patient suffering from an inflammatory disease, the TNF-α amount in plasma of the patient is measured. As a result, the TNF-α amount decreases from that before the administration.

### Industrial Applicability

The TNF-α inhibitor of the present invention has a superior TNF-α inhibitory effect and is useful as an agent for the prophylaxis or treatment of diseases in which TNF-α is involved, such as an inflammatory disease and the like.

## Claims

1. A TNF-α inhibitor comprising an insulin sensitizer in combination with an HMG-CoA reductase inhibitor.

2. The inhibitor of claim 1, wherein the insulin sensitizer is a compound of the formula [I] 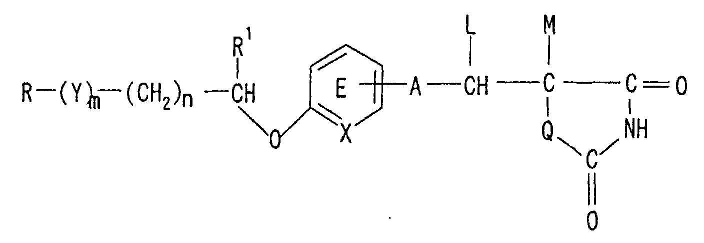 wherein
R is an optionally substituted hydrocarbon group or an optionally substituted heterocyclic group;
Y is a group represented by -CO-, -CH(OH)- or -NR³- wherein R³ is an optionally substituted alkyl group;
m is 0 or 1;
n is 0, 1 or 2;
X is CH or N;
A is a bond or a divalent aliphatic hydrocarbon group having 1 to 7 carbon atoms;
Q is an oxygen atom or a sulfur atom;
R¹ is a hydrogen atom or an alkyl group;
ring E may further have 1 to 4 substituents, wherein the substituent(s) may be bonded to R¹ to form a ring; and
L and M are each a hydrogen atom or may form a bond in combination,
or a salt thereof.

3. The inhibitor of claim 2, wherein the compound of the formula [I] is pioglitazone.

4. The inhibitor of claim 2, wherein the compound of the formula [I] is rosiglitazone.

5. The inhibitor of claim 1, wherein the HMG-CoA reductase inhibitor is a statin compound.

6. The inhibitor of claim 1, wherein the HMG-CoA reductase inhibitor is cerivastatin, pravastatin, simvastatin, lovastatin, atorvastatin, fluvastatin, itavastatin, ZD-4522 or a salt thereof.

7. The inhibitor of claim 1, wherein the HMG-CoA reductase inhibitor is atorvastatin or a salt thereof.

8. The inhibitor of claim 1, which comprises pioglitazone or a salt thereof in combination with cerivastatin or a salt thereof.

9. The inhibitor of claim 1, which comprises pioglitazone or a salt thereof in combination with pravastatin or a salt thereof.

10. The inhibitor of claim 1, which comprises pioglitazone or a salt thereof in combination with atorvastatin or a salt thereof.

11. The inhibitor of claim 1, which comprises rosiglitazone or a salt thereof in combination with cerivastatin or a salt thereof.

12. The inhibitor of claim 1, which comprises rosiglitazone or a salt thereof in combination with pravastatin or a salt thereof.

13. The inhibitor of claim 1, which comprises rosiglitazone or a salt thereof in combination with atorvastatin or a salt thereof.

14. The inhibitor of claim 1, which is an agent for the prophylaxis or treatment of an inflammatory disease.

15. The inhibitor of claim 14, wherein the inflammatory disease is rheumatoid arthritis.

16. The inhibitor of claim 14, wherein the inflammatory disease is inflammatory bowel disease.

17. A method for treating an inflammatory disease, which comprises administering an effective amount of an insulin sensitizer in combination with an HMG-CoA reductase inhibitor to a mammal.

18. Use of an insulin sensitizer for the production of a therapeutic agent of an inflammatory disease which is used in combination with an HMG-CoA reductase inhibitor.
